(19) Europäisches Patentamt / European Patent Office / Office européen des brevets

(11) **EP 2 868 260 A1**

(12) **EUROPEAN PATENT APPLICATION**
published in accordance with Art. 153(4) EPC

(43) Date of publication:
**06.05.2015 Bulletin 2015/19**

(51) Int Cl.:
***A61B 5/00*** *(2006.01)*

(21) Application number: **12880042.2**

(86) International application number:
**PCT/JP2012/066722**

(22) Date of filing: **29.06.2012**

(87) International publication number:
**WO 2014/002255 (03.01.2014 Gazette 2014/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Kabushiki Kaisha Toshiba**
**Minato-ku**
**Tokyo 105-8001 (JP)**

(72) Inventor: **MURAKAMI, Tomoko**
**Minato-ku**
**Tokyo**
**105-8001 (JP)**

(74) Representative: **Granleese, Rhian Jane**
**Marks & Clerk LLP**
**90 Long Acre**
**London WC2E 9RA (GB)**

(54) **HEALTH MANAGEMENT SUPPORT DEVICE, METHOD AND PROGRAM**

(57) According to an embodiment, there is provided a healthcare support apparatus including a shooting means for shooting a skin image of a subject, a calculation means for obtaining a luminance value of a first color and a luminance value of a second color from the skin image, converting the luminance value of the first color and the luminance value of the second color into absorbances, and calculating a hemoglobin content from the absorbances, an accumulation means for accumulating a first history of the hemoglobin content and a second history of a living event of the subject, a living behavior identification means for determining a significance of a tendency of the hemoglobin content based on the first history and identifying a living behavior corresponding to the significance based on the second history, and an output means for outputting information indicating the living behavior.

FIG. 1

**Description**

Technical Field

Embodiments of the present invention relate to a healthcare support apparatus, method, and program, which support healthcare on the basis of measurement of blood circulation.

Background Art

Regarding daily healthcare support, an apparatus that supports healthcare by utilizing the measurements or recorded information such as bioinstrumentation (for example, measurement of a body temperature, body weight, or blood circulation of a person), behavior measure (for example, measurement of the number of steps, or amount of activity), and a record relating to contents of meals is already provided.

In healthcare support based on bioinstrumentation, particularly based on measurement of a blood circulation state, provision of an apparatus which requires no blood sampling, and can be utilized by anyone in the home is desired.

Citation List

Patent Literature

Patent Literature 1: Jpn. Pat. Appln. KOKAI Publication No. 10-127585

Non-Patent Literature

Non-Patent Literature 1: The Japanese Journal of Dermatology: 105 (1), 43-49, 1995

Summary of Invention

Technical Problem

Provision of a healthcare support apparatus, method, and program requiring no massive measurement equipment, and capable of simply and easily measuring a blood circulation state without burdening the subject is desired.

Solution to Problem

According to an embodiment, there is provided a healthcare support apparatus including a shooting means for shooting a skin image of a subject, a calculation means for obtaining a luminance value of a first color and a luminance value of a second color from the skin image, converting the luminance value of the first color and the luminance value of the second color into absorbances, and calculating a hemoglobin content from the absorbances, an accumulation means for accumulating a first history of the hemoglobin content and a second history of a living event of the subject, a living behavior identification means for determining a significance of a tendency of the hemoglobin content based on the first history and identifying a living behavior corresponding to the significance based on the second history, and an output means for outputting information indicating the living behavior.

Brief Description of Drawings

FIG. 1 is a block diagram showing a healthcare support apparatus according to an embodiment.
FIG. 2 is a view for explaining a shooting unit and an output unit.
FIG. 3 is a flowchart showing calculation processing of an erythema index.
FIG. 4 is a flowchart showing living behavior identification processing.
FIG. 5 is a view showing examples of the rule for identifying the living behavior.
FIG. 6 is a view showing an example of input of living behavior information.
FIG. 7 is a view showing an example of living behavior history data.
FIG. 8 is a view showing an example of an output screen of the healthcare support apparatus.
FIG. 9 is a view showing a result of a measurement experiment using the healthcare support apparatus.
FIG. 10 is a graph showing a relationship between the wavelength of light and an absorption coefficient.

Description of Embodiments

Hereinafter, an embodiment will be described with reference to the drawings. The embodiment relates to a healthcare support apparatus utilizable even in, for example, a home, and based on blood circulation measurement. This apparatus irradiates the skin of a subject with light, and measures the blood circulation of the subject by image processing of the shot image (called a "skin image"). More specifically, an erythema index proportional to the hemoglobin content is calculated from the shot skin image as a degree of smoothness of blood circulation, and the calculated erythema index is presented as a measurement result. Further, this apparatus accumulates the history of information items on the erythema indices obtained by such blood circulation measurement, and the history of living event information items on sports, bathing, and the like. A living behavior effective for blood circulation improvement is identified based on the accumulated information items, and is output as information for supporting healthcare.

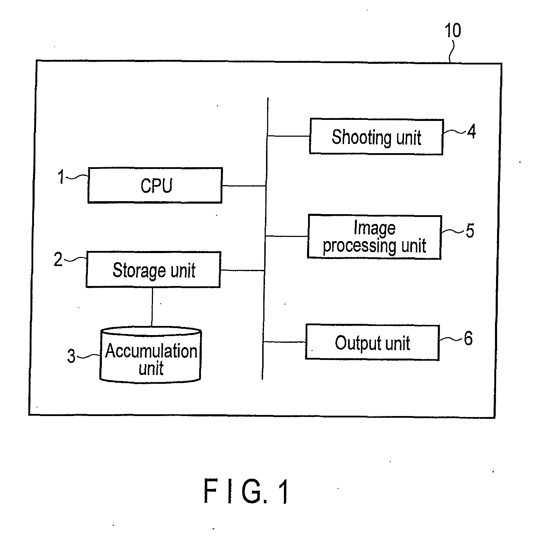

FIG. 1 shows a block diagram of a healthcare support apparatus according to an embodiment. The healthcare support apparatus 10 includes a CPU 1, a storage unit 2, an accumulation unit 3, a shooting unit 4, an image processing unit 5, and an output unit 6.

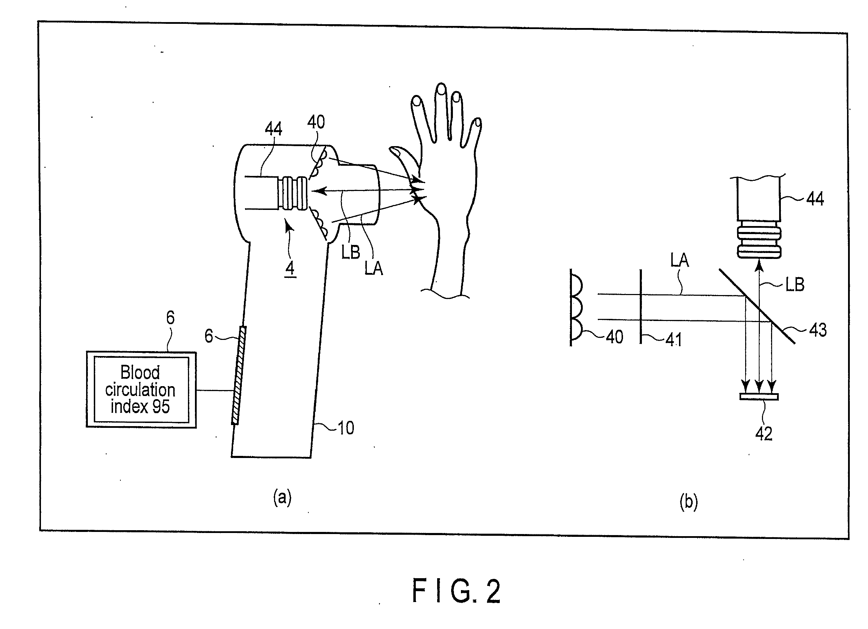

The healthcare support apparatus 10 according to the present embodiment is realized as, for example, a handheld device. As shown in (a) of FIG. 2, the healthcare support apparatus 10 can be held by a hand of a person to be used. In (a) of FIG. 2, a state where the healthcare support apparatus 10 is directed to the back of a hand of a subject, thereby carrying out blood circulation measurement is shown. As can be seen from (a) of FIG. 2, blood circulation measurement can be carried out without contact with the subject and, as a result of the measurement, for example, a "blood circulation index 95" is displayed on the output unit 6. This numerical value is a value corresponding to the erythema index, and the manner, and the like in which the numerical value is calculated based on the shot skin image will be described later in detail.

Further, on the output unit 6, not only the blood circulation index (erythema index), but also information for supporting healthcare of the subject is displayed. This information corresponds to a result of identification (estimation) of a living behavior substantiating an improvement tendency or a deterioration tendency of blood circulation, and is displayed together with information on a blood circulation measurement result. This point will also be described later in detail.

The configuration of (a) of FIG. 2 corresponds to a configuration of the healthcare support apparatus 10 in which all the elements shown in FIG. 1 are included. A charge-coupled device (CCD) camera 44 and white light-emitting diode (LED) 40 are incorporated in the apparatus as the shooting unit 4, and a display is provided in the apparatus as the output unit 6. Part of the elements shown in FIG. 1 may be made external units, and a signal may be transmitted between the units by wire or wireless.

(b) of FIG. 2 shows a configuration example of an optical system of the shooting unit 4. Light LA emitted from the white LED 40 passes through a light blocking plate 41 which blocks light from the opposite side of the white LED 40, is reflected from a half mirror 43, and is incident on an object 42 to be shot. In the case of this example, the object 42 to be shot is the back of the hand of the subject. Part LB of the light reflected from the object 42 passes through the half mirror 43, and is incident on the CCD camera 44. Part of the light reflected from the object 42 and reflected from the half mirror 43 is blocked by the light blocking plate 41, and is not incident on the white LED 40 side.

The CCD camera 44 carries out a shooting operation for the incident light LB, and shoots (takes) the image of the object 42, i.e., the skin image of the back of the hand of the subject in this example. It should be noted that shooting of the skin image may be carried out by an operation or the like of releasing a measurement execution button (not shown) to be done by the subject or the like.

It should be noted that a complementary metal-oxide semiconductor (CMOS) camera may be used as the shooting unit 4 in place of the CCD camera 44. Further, the light source configured to irradiate the shooting-target area of the subject with light is not limited to the white LED, and a light source of a different type such as a light source of trichromatic light may be used. Alternatively, a configuration in which the skin image is shot under natural light without providing a light source configured to irradiate the shooting-target area with light may be employed.

The configuration of the healthcare support apparatus 10 is not limited to the configuration shown in FIG. 2. Those skilled in the art can variously modify the present embodiment to apply blood circulation measurement and healthcare support to other devices and instruments.

The function of blood circulation measurement and healthcare support based on the shot skin image may be realized by computer software. In this case, a program causing a computer to function as the healthcare support apparatus according to the present embodiment is recorded on a recording medium (not shown) or the like, the program is read into the storage unit 2, and is executed by the CPU 1.

In the healthcare support apparatus 10, the image processing unit 5 obtains a luminance value of a first color and a luminance value of a second color from the skin image shot by the shooting unit 4, separately converts the luminance value of the first color and the luminance value of the second color into absorbances, and calculates the hemoglobin content from the two absorbances. For example, the hemoglobin content described above is an erythema index based on the two absorbances.

In the present embodiment, the first color is, for example, red of the three primary colors of light, and the second color is, for example, green of the three primary colors of light. In this case, the image processing unit 5 calculates a first reflectance from the luminance value of red of the three primary colors in the skin image, and calculates a first absorbance from the first reflectance. Further, the image processing unit 5 calculates a second reflectance from the luminance value of green of the three primary colors in the skin image, and calculates a second absorbance from the second reflectance. Further, the image processing unit 5 calculates an erythema index based on the first absorbance and the second absorbance.

The accumulation unit 3 accumulates therein a first history of the hemoglobin content calculated by the image processing unit 5. Further, the accumulation unit 3 also accumulates therein a second history of the living event of the subject.

The CPU 1 determines a significance of the tendency of the hemoglobin content based on the first history of the hemoglobin content accumulated in the accumulation unit 3, and identifies a living behavior corresponding to the significance based on the second history of the living event of the subject.

The output unit 6 outputs information indicating the living behavior corresponding to the significance of the tendency of the hemoglobin content identified by the CPU 1.

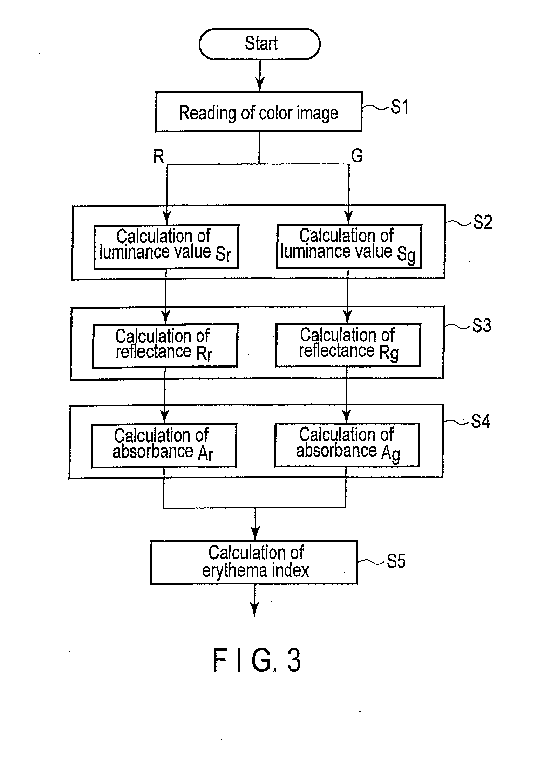

The calculation processing of the erythema index for blood circulation measurement according to the present embodiment will be described below while referring to the flowchart of FIG. 3.

The shooting unit 4 shoots a color image of the skin under irradiation of the white LED polarized light shown in FIG. 2. The image processing unit 5 reads the shot color image (S1), and calculates luminance values Sr and Sg for respective channels of the three primary colors (RGB) of light (S2). The luminance value Sr corresponds to red of the three primary colors of light, and the luminance value Sg corresponds to green of the three primary colors of light. For example, each of the luminance values Sr and Sg may be made an average value of the luminance values of corresponding pixels. That is, an average value of the luminance values of red pixels may be made the luminance value Sr, and an average value of the luminance values of green pixels may be made the luminance value Sg.

Next, the image processing unit 5 calculates a reflectance Rr and reflectance Rg according to the following formula (1) (S3).

$$Rr=Sr/255; \quad Rg=Sg/255 \quad \cdots \quad (1)$$

In the present embodiment, the above reflectance values are made luminance values (Sr, Sg) of light reflected from a certain surface relative to the luminance value of light reflected from a white surface. The luminance value of light reflected from a white surface is, for example, "255". The reflectance ranges from 0 to 1. When the reflectance is large, it approaches 1 and, when the reflectance is small, it approaches 0.

Next, the image processing unit 5 calculates absorbances Ar and Ag according to the following formula (2) (S4).

$$Ar=-\log_{10}Rr; \quad Ag=-\log_{10}Rg \quad \cdots \quad (2)$$

Further, the image processing unit 5 calculates an erythema index EI according to the following formula (3) (S5).

$$EI=Ag-\alpha Ar \quad \cdots \quad (3)$$

The erythema index EI is hardly affected by melanin, and is approximately proportional to the hemoglobin content. In the present embodiment, the erythema index EI calculated by the image processing unit 5 is made a barometer indicating the degree of smoothness of blood circulation.

Here, a correction coefficient $\alpha$ is a coefficient for eliminating an influence of the melanin content, and an average value of $\alpha$ of Japanese people is about 0.14 ($\alpha$=0.14).

A result of measurement of blood circulation obtained as an erythema index EI based on the image processing is accumulated in the accumulation unit 3 through the storage unit 2.

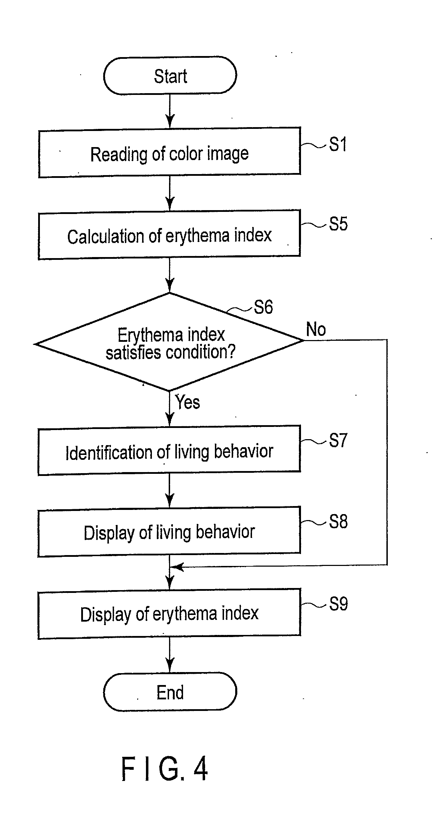

The living behavior identification processing based on the blood circulation measurement result according to the present embodiment will be described below while referring to the flowchart of FIG. 4.

When the erythema index is calculated by the image processing unit 5 in the processing shown in FIG. 3 (S5), the storage unit 2 reads the erythema index history information accumulated in the accumulation unit 3. The CPU 1 determines whether or not the tendency of the erythema index is significant in the read erythema index history (S6).

Regarding the significance of the tendency of the erythema index, setting of various evaluation scales such as an improvement tendency of blood circulation, deterioration tendency thereof, and the like is conceivable. Further, determination of the significance of the tendency of the erythema index may be carried out by threshold processing. For example, a threshold of the erythema index is set in advance as a determination condition for the improvement tendency of blood circulation. For example, when "the erythema index is greater than or equal to 100 continuously for the past three days" holds, it is determined that there is a significance in the tendency of the erythema index. Further, a rate of change indicating a fact or the like that "the average value of the erythema indices of this week is 1.1 times greater than the average value of one week ago" may be made a condition.

If it is determined that the tendency of the erythema index satisfies the condition, and is significant (S6), then a living behavior associated with the significance of the tendency of the erythema index is identified (S7).

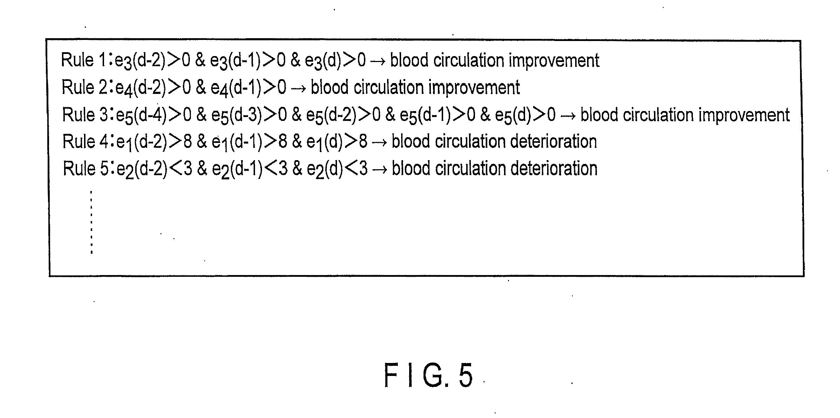

More specifically, the CPU 1 applies a rule shown in, for example, FIG. 5 to the history of the living event of the subject accumulated in the accumulation unit 3 to thereby identify the living behavior corresponding to the significance of the tendency of the erythema index determined in step (S6).

The "rule 1" of FIG. 5 is a rule of considering that blood circulation improvement has been affected when the number of times $e_3(d)$ of bathing $e_3$ of the measurement day (d) is greater than or equal to 1, the number of times $e_3(d-1)$ of bathing $e_3$ of the day (d-1) before the measurement day is greater than or equal to 1, and the number of times $e_3(d-2)$ of bathing $e_3$ of the day (d-2) two days before the measurement day is greater than or equal to 1, i.e., when recording of bathing for 3 consecutive days including the measurement day is confirmed. The CPU 1 identifies a living behavior conforming to the rule by referring to the rule for determining such blood circulation improvement or blood circulation deterioration, and living behavior history information (S7). Identification of a living behavior corresponding to the significant tendency of the erythema index is not limited to that based on each of the rules shown in FIG. 5. Identification of a living behavior may be made on the basis of information of other forms such as a model, numerical formula or the like in place of such a rule.

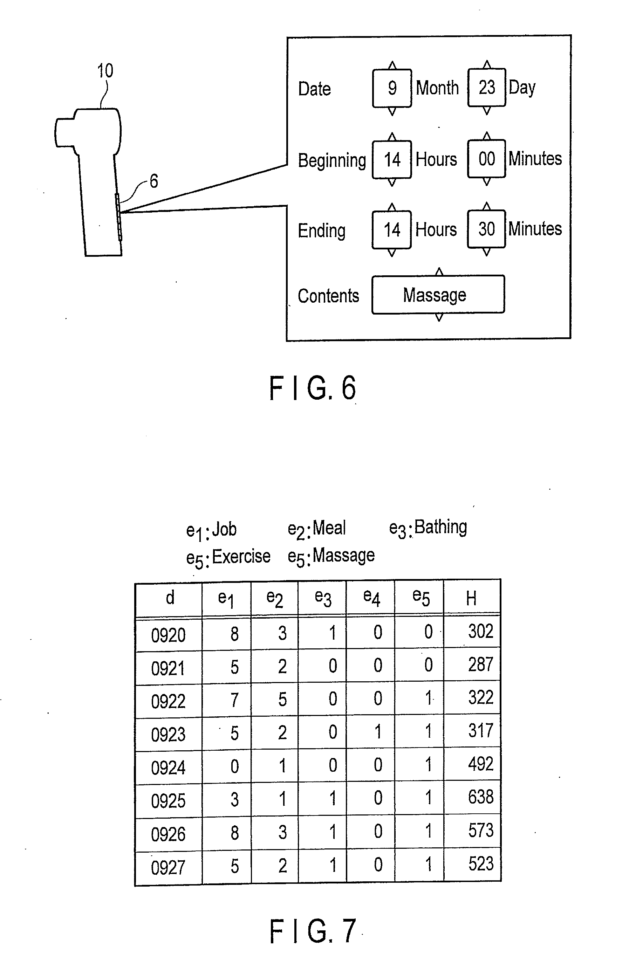

As the living behavior information, the user (mainly the subject) inputs information such as a date, beginning time, ending time, contents of a living event (example: massage), and the like on the same display of the output unit 6. It should be noted that the user may input living behavior information through, for example, a device connected to a network or input living behavior information by utilizing some other type of medium instead of utilizing the input interface shown in FIG. 6.

The input living behavior information on the user is accumulated in the accumulation unit 3 through the storage unit 2 as living behavior history information as shown in FIG. 7. For example, attention is paid to September 27 (d=0927). When the rule of FIG. 5 is applied to the living behavior history data of FIG. 7, bathing is done from September 25 to 27, and hence the "rule 1" is satisfied, and massage is carried out from September 23 to September 27, and hence the "rule 3" is satisfied.

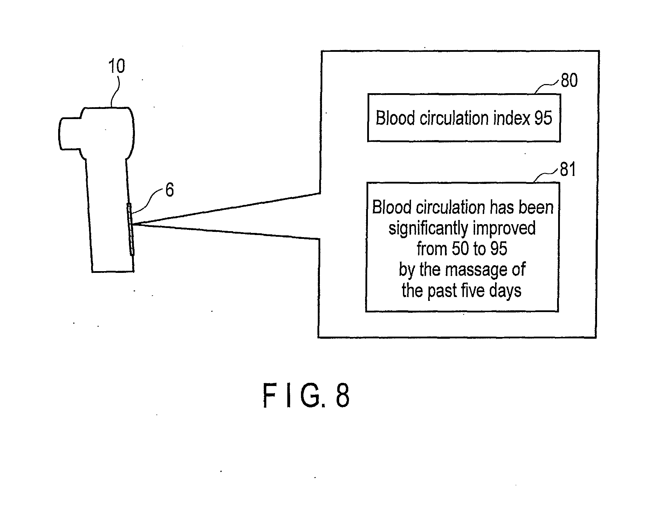

The output unit 6 displays information 81 indicating the identified living behavior (S8), and also displays the erythema index (a blood circulation index "95" in this example) (S9) as shown in FIG. 8. When it is determined that the tendency of the erythema index described above does not satisfy the condition, and is not significant (S6), the output unit 6 displays only information 80 indicating the erythema index (S9).

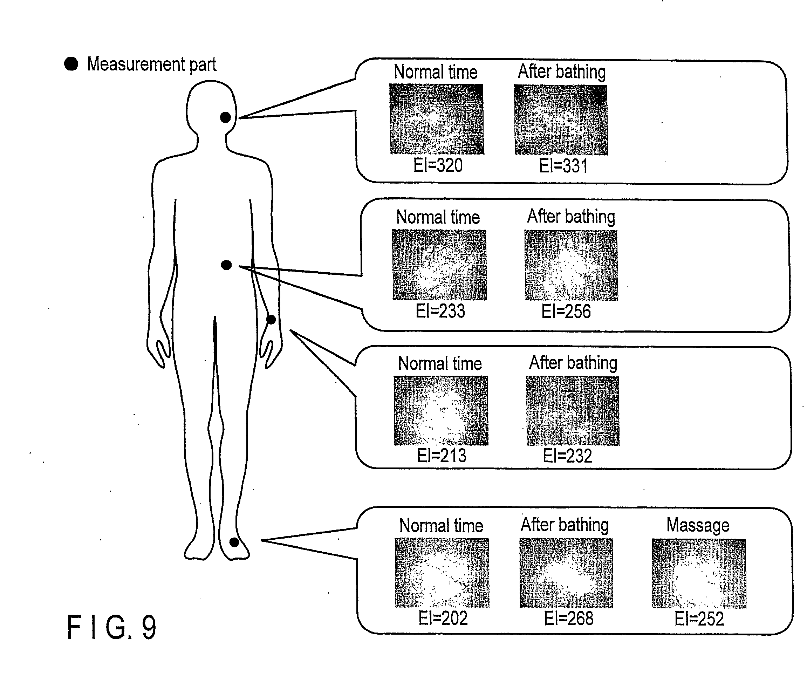

FIG. 9 shows a result of a measurement experiment using the healthcare support apparatus 10 according to the present embodiment. By measuring the blood circulation state at a normal time, after bathing, and after massage with respect to four parts including the cheek, abdomen, outside wrist, and toes, it has been found that the value of the erythema index EI becomes higher at each part. Particularly, even at parts of the body such as the cheek, and outside wrist at which the influence of melanin is strong, it has been possible to confirm improvement in the blood circulation state.

According to the embodiment described above, it is possible to provide a healthcare support apparatus, method, and program requiring no massive measurement equipment, and capable of simply and easily measuring the blood circulation state without burdening the subject.

Accordingly, it becomes possible for persons in a home to simply and easily measure blood circulation at an arbitrary part of the body, learn a living behavior affecting the blood circulation state, and effectively support healthcare. As is stated that measurement can be performed simply and easily, the healthcare support apparatus according to the embodiment does not essentially require a specific technique or a specific medicine such as blood sampling or application of gel which has been required in the conventional measurement devices and instruments.

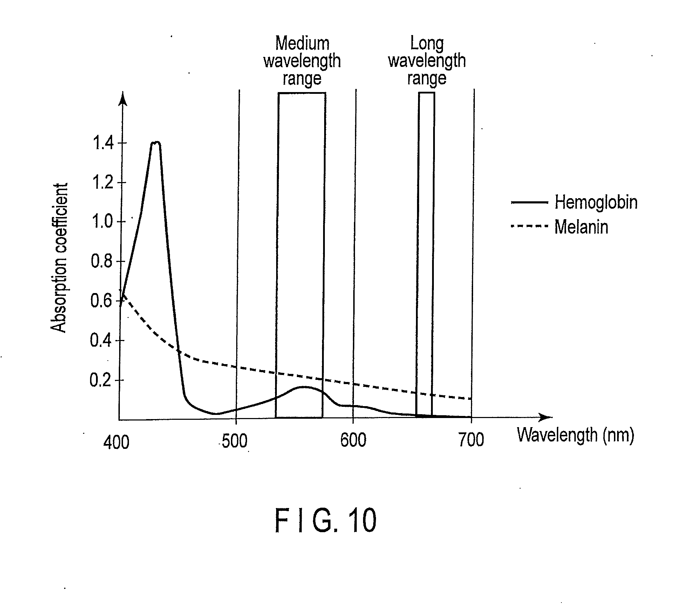

FIG. 10 is a graph showing a relationship between the wavelength of light and absorption coefficient with respect to hemoglobin and melanin. The solid line indicates the absorption coefficient of hemoglobin, and the dotted line indicates the absorption coefficient of melanin. In the case not according to the present embodiment, it is possible to select two wavelengths, including a wavelength in the medium wavelength (540 to 570 nm) range and a long wavelength (660 nm) at which hemoglobin is less affected, and make a difference between the absorption coefficients at both the wavelengths a measurement result of blood circulation. In this case, relatively massive measurement equipment including a spectrometer is required. However, according to the present embodiment, it is possible to realize the function of blood circulation measurement simply and at low cost without the need for such massive measurement equipment.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

**Claims**

1.  A healthcare support apparatus comprising:

    a shooting means for shooting a skin image of a subject;
    a calculation means for obtaining a luminance value of a first color and a luminance value of a second color from the skin image, converting the luminance value of the first color and the luminance value of the second color into absorbances, and calculating a hemoglobin content from the absorbances;
    an accumulation means for accumulating a first history of the hemoglobin content and a second history of a living event of the subject;
    a living behavior identification means for determining a significance of a tendency of the hemoglobin content based on the first history and identifying a living behavior corresponding to the significance based on the second history; and
    an output means for outputting information indicating the living behavior.

2.  The apparatus according to claim 1, wherein the hemoglobin content is an erythema index based on the absorbances.

3.  The apparatus according to claim 2, wherein
    the calculation means
    calculates a first reflectance from a luminance value of red of three primary colors in the skin image and calculates a first absorbance from the first reflectance,
    calculates a second reflectance from a luminance value of green of the three primary colors in the skin image and calculates a second absorbance from the second reflectance, and
    calculates the erythema index based on the first absorbance and the second absorbance.

4.  The apparatus according to claim 1, wherein the significance of the tendency of the hemoglobin content indicates an improvement tendency or a deterioration tendency of blood circulation.

5.  The apparatus according to claim 1, further comprising means for setting a threshold for the hemoglobin content, wherein
    the living behavior identification means determines the significance of the tendency of the hemoglobin content based on the threshold.

6.  A healthcare support method comprising:

    a step of shooting a skin image of a subject;
    a step of obtaining a luminance value of a first color and a luminance value of a second color from the skin image, converting the luminance value of the first color and the luminance value of the second color into absorbances, and calculating a hemoglobin content from the absorbances;
    a step of accumulating a first history of the hemoglobin content and a second history of a living event of the subject;
    a step of determining a significance of a tendency of the hemoglobin content based on the first history and identifying a living behavior corresponding to the significance based on the second history; and
    a step of outputting information indicating the living behavior.

7.  A healthcare support program causing a computer to function as
    a calculation means for obtaining a luminance value of a first color and a luminance value of a second color from a skin image of a subject, converting the luminance value of the first color and the luminance value of the second color into absorbances, and calculating a hemoglobin content from the absorbances;
    an accumulation means for accumulating a first history of the hemoglobin content and a second history of a living event of the subject;

a living behavior identification means for determining a significance of a tendency of the hemoglobin content based on the first history and identifying a living behavior corresponding to the significance based on the second history; and an output means for outputting information indicating the living behavior.

10

1 — CPU

2 — Storage unit

3 — Accumulation unit

4 — Shooting unit

5 — Image processing unit

6 — Output unit

# FIG. 1

F I G. 2

Start

Reading of color image ～S1

R          G

Calculation of
luminance value $S_r$          Calculation of
luminance value $S_g$          ～S2

Calculation of
reflectance $R_r$          Calculation of
reflectance $R_g$          ～S3

Calculation of
absorbance $A_r$          Calculation of
absorbance $A_g$          ～S4

Calculation of
erythema index          ～S5

F I G. 3

```
                         ╭─────────────╮
                         │    Start    │
                         ╰──────┬──────╯
                                │
                                ▼
              ┌──────────────────────────────┐
              │    Reading of color image    │───S1
              └───────────────┬──────────────┘
                              │
                              ▼
              ┌──────────────────────────────┐
              │ Calculation of erythema index │───S5
              └───────────────┬──────────────┘
                              │
                              ▼
                          ╱───────╲               S6
                        ╱           ╲
                      ╱  Erythema index ╲      No
                     ⟨  satisfies condition? ⟩──────┐
                      ╲               ╱            │
                        ╲           ╱              │
                          ╲───────╱                │
                              │ Yes                │
                              ▼                    │
              ┌──────────────────────────────┐     │
              │ Identification of living behavior │───S7 │
              └───────────────┬──────────────┘     │
                              │                    │
                              ▼                    │
              ┌──────────────────────────────┐     │
              │   Display of living behavior   │───S8 │
              └───────────────┬──────────────┘     │
                              │◄───────────────────┘
                              ▼
              ┌──────────────────────────────┐
              │   Display of erythema index   │───S9
              └───────────────┬──────────────┘
                              │
                              ▼
                         ╭─────────────╮
                         │     End     │
                         ╰─────────────╯
```

F I G. 4

EP 2 868 260 A1

Rule 1: $e_3(d-2) > 0$ & $e_3(d-1) > 0$ & $e_3(d) > 0 \rightarrow$ blood circulation improvement

Rule 2: $e_4(d-2) > 0$ & $e_4(d-1) > 0 \rightarrow$ blood circulation improvement

Rule 3: $e_5(d-4) > 0$ & $e_5(d-3) > 0$ & $e_5(d-2) > 0$ & $e_5(d-1) > 0$ & $e_5(d) > 0 \rightarrow$ blood circulation improvement

Rule 4: $e_1(d-2) > 8$ & $e_1(d-1) > 8$ & $e_1(d) > 8 \rightarrow$ blood circulation deterioration

Rule 5: $e_2(d-2) < 3$ & $e_2(d-1) < 3$ & $e_2(d) < 3 \rightarrow$ blood circulation deterioration

⋮

F I G. 5

Date [9] Month [23] Day

Beginning [14] Hours [00] Minutes

Ending [14] Hours [30] Minutes

Contents [Massage]

F I G. 6

$e_1$:Job     $e_2$:Meal     $e_3$:Bathing
$e_5$:Exercise   $e_5$:Massage

| d | $e_1$ | $e_2$ | $e_3$ | $e_4$ | $e_5$ | H |
|------|------|------|------|------|------|------|
| 0920 | 8 | 3 | 1 | 0 | 0 | 302 |
| 0921 | 5 | 2 | 0 | 0 | 0 | 287 |
| 0922 | 7 | 5 | 0 | 0 | 1 | 322 |
| 0923 | 5 | 2 | 0 | 1 | 1 | 317 |
| 0924 | 0 | 1 | 0 | 0 | 1 | 492 |
| 0925 | 3 | 1 | 1 | 0 | 1 | 638 |
| 0926 | 8 | 3 | 1 | 0 | 1 | 573 |
| 0927 | 5 | 2 | 1 | 0 | 1 | 523 |

F I G. 7

10

6

80

Blood circulation index 95

81

Blood circulation has been
significantly improved
from 50 to 95
by the massage of
the past five days

FIG. 8

FIG. 9

FIG. 10

<table>
<tr><td colspan="2">**INTERNATIONAL SEARCH REPORT**</td><td>International application No.<br>PCT/JP2012/066722</td></tr>
</table>

A. CLASSIFICATION OF SUBJECT MATTER
*A61B5/00*(2006.01)i

According to International Patent Classification (IPC) or to both national classification and IPC

B. FIELDS SEARCHED

Minimum documentation searched (classification system followed by classification symbols)
A61B5/00

Documentation searched other than minimum documentation to the extent that such documents are included in the fields searched
| Jitsuyo Shinan Koho | 1922–1996 | Jitsuyo Shinan Toroku Koho | 1996–2012 |
| Kokai Jitsuyo Shinan Koho | 1971–2012 | Toroku Jitsuyo Shinan Koho | 1994–2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2010-227571 A (Johnson & Johnson Consumer Companies, Inc.), 14 October 2010 (14.10.2010), paragraphs [0020] to [0023] & US 2010/0249731 A1 & EP 2233073 A1 & CN 101843475 A & CA 2697316 A & AU 2010201207 A & KR 10-2010-0108280 A & RU 2010111626 A | 1-7 |
| Y | JP 10-057320 A (Nippon Zeon Co., Ltd.), 03 March 1998 (03.03.1998), paragraph [0018] (Family: none) | 1-7 |

[X] Further documents are listed in the continuation of Box C.  [ ] See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 09 August, 2012 (09.08.12) | 21 August, 2012 (21.08.12) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
| --- |
| PCT/JP2012/066722 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2007-307084 A  (Shiseido Co., Ltd.), 29 November 2007 (29.11.2007), paragraphs [0006], [0009] to [0012] (Family: none) | 4,5 |

Form PCT/ISA/210 (continuation of second sheet) (July 2009)

**EP 2 868 260 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10127585 A

**Non-patent literature cited in the description**

- *The Japanese Journal of Dermatology,* 1995, vol. 105 (1), 43-49